# EUROPÄISCHE PATENTSCHRIFT

(11) **EP 1 592 671 B1**
(45) Veröffentlichungstag und Bekanntmachung des Hinweises auf die Patenterteilung: **09.04.2008**
(21) Anmeldenummer: 04703374.1
(22) Anmeldetag: 20.01.2004
(51) Int. Cl.: C07D 235/30, A61K 31/4184, A61P 11/00

(54) **N-SUBSTITUIERTE (BENZOIMIDAZOL-2-YL)-PHENYL-AMINE, VERFAHREN ZU IHRER HERSTELLUNG, IHRE VERWENDUNG ALS MEDIKAMENT ODER DIAGNOSTIKUM SOWIE SIE ENTHALTENDES MEDIKAMENT**
N-SUBSTITUTED (BENZOIMIDAZOLE-2-YL) PHENYL AMINES, METHOD FOR THEIR PRODUCTION, THEIR USE AS A MEDICAMENT OR DIAGNOSTIC AGENT AND MEDICAMENT CONTAINING SAID SUBSTANCES
(BENZOIMIDAZOL-2-YL)-PHENYLAMINES N-SUBSTITUEES, LEUR PROCEDE DE PRODUCTION, LEUR UTILISATION COMME MEDICAMENT OU AGENT DIAGNOSTIC, ET MEDICAMENT LES CONTENANT

(30) Priorität: 04.02.2003 DE 10304294
(43) Veröffentlichungstag der Anmeldung: 09.11.2005
(73) Patentinhaber: Sanofi-Aventis Deutschland GmbH, 65929 Frankfurt am Main (DE)
(72) Erfinder: HEINELT, Uwe, 65187 Wiesbaden (DE); LANG, Hans-Jochen, 65719 Hofheim (DE); HOFMEISTER, Armin, 55276 Oppenheim (DE); WIRTH, Klaus, 65830 Kriftel (DE); HUG, Martin, 79117 Freiburg (DE)
(86) Internationale Anmeldenummer: PCT/EP2004/000395
(87) Internationale Veröffentlichungsnummer: WO 2004/069811

(56) Entgegenhaltungen:
- WO-A-02/46169

## Beschreibung

Die Erfindung betrifft Verbindungen vom Typ der N-substituierten (Benzoimidazol-2-yl)-phenyl-amine, die den Natrium-Protonen-Austauscher Subtyp 3 (NHE3) hemmen und die nützlich bei der Prävention oder Behandlung diverser Erkrankungen sind. So lassen sich die Verbindungen unter anderem bei Nierenerkrankungen wie akutem oder chronischem Nierenversagen, bei Störungen der Gallenfunktion, bei Atemstörungen wie Schnarchen oder Schlafapnoen oder bei Schlaganfall einsetzen.

Die Erfindung betrifft Verbindungen der Formel I, 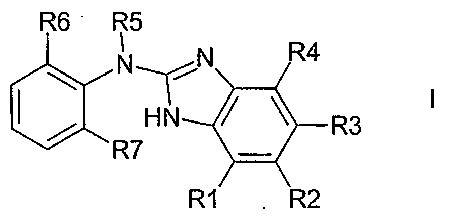 worin bedeuten:
- R1 und R4: unabhängig voneinander H, F, Cl, Br, Alkyl mit 1, 2, 3 oder 4 C-Atomen oder Alkoxy mit 1, 2, 3 oder 4 C-Atomen,
wobei die Alkyl- und Alkoxygruppen unsubstituiert sind oder unabhängig voneinander mit 1, 2, 3, 4, 5, 6, 7, 8 oder 9 F-Atomen substituiert sind;
- R2 und R3: unabhängig voneinander H, F, Alkyl mit 1, 2 oder 3 C-Atomen, Alkoxy mit 1, 2 oder 3 C-Atomen oder OH,
wobei die Alkyl- und Alkoxygruppen unsubstituiert sind oder unabhängig voneinander mit 1, 2, 3, 4, 5, 6 oder 7 F-Atomen substituiert sind;
- R5: Alkyl mit 1, 2, 3 oder 4 C-Atomen, Alkenyl mit 2, 3 oder 4 C-Atomen oder Cycloalkyl mit 3, 4 oder 5 C-Atomen,
wobei die Alkyl-, Alkenyl-, und Cycloalkylgruppen unsubstituiert sind oder mit 1, 2, 3, 4, 5, 6, 7, 8 oder 9 F-Atomen substituiert sind;
- R6 und R7: unabhängig voneinander H, F, Cl, Br, Alkyl mit 1, 2, 3 oder 4 C-Atomen oder Alkoxy mit 1, 2 oder 3 C-Atomen,
wobei die Alkyl- und Alkoxygruppen unsubstituiert sind oder unabhängig voneinander mit 1, 2, 3, 4, 5, 6, 7, 8 oder 9 F-Atomen substituiert sind,
und wenigstens einer der beiden Reste, R6 oder R7, nicht Wasserstoff entspricht;
sowie deren pharmazeutisch verträgliche Salze und Trifluoressigsäure-salze.

Bevorzugt sind Verbindungen der Formel 1, worin bedeuten:
- R1 und R4: unabhängig voneinander H, F, Cl, Br, Alkyl mit 1, 2, 3 oder 4 C-Atomen oder Alkoxy mit 1, 2, 3 oder 4 C-Atomen,
wobei die Alkyl- und Alkoxygruppen unsubstituiert sind oder unabhängig voneinander mit 1, 2, 3, 4, 5, 6, 7, 8 oder 9 F-Atomen substituiert sind;
- R2 und R3: unabhängig voneinander H, F, Alkyl mit 1, 2 oder 3 C-Atomen, Alkoxy mit 1, 2 oder 3 C-Atomen oder OH,
wobei die Alkyl- und Alkoxygruppen unsubstituiert sind oder unabhängig voneinander mit 1, 2, 3, 4, 5, 6 oder 7 F-Atomen substituiert sind;
- R5: Alkyl mit 1, 2, 3 oder 4 C-Atomen, Alkenyl mit 2, 3 oder 4 C-Atomen oder Cycloalkyl mit 3, 4 oder 5 C-Atomen,
wobei die Alkyl-, Alkenyl-, und Cycloalkylgruppen unsubstituiert sind oder mit 1, 2, 3, 4, 5, 6, 7, 8 oder 9 F-Atomen substituiert sind;
- R6 und R7: unabhängig voneinander F, Cl, Br, Alkyl mit 1, 2 oder 3 C-Atomen oder Alkoxy mit 1, 2 oder 3 C-Atomen,
wobei die Alkyl- und Alkoxygruppen unsubstituiert sind oder unabhängig voneinander mit 1, 2, 3, 4, 5, 6 oder 7 F-Atomen substituiert sind;
und wenigstens einer der beiden Reste, R6 oder R7, nicht Wasserstoff entspricht;
sowie deren pharmazeutisch verträgliche Salze und Trifluoressigsäure-salze.

In einer Ausführungsform sind Verbindungen der Formel I bevorzugt, in denen R1 und R4 unabhängig voneinander durch H oder F beschrieben werden. In einer weiteren Ausführungsform sind Verbindungen der Formel I bevorzugt, in denen R2 und R3 unabhängig voneinander durch H oder F beschrieben werden. In einer weiteren Ausführungsform sind Verbindungen der Formel I bevorzugt, in denen R5 durch Methyl, Ethyl, Isopropyl, Allyl öder Cyclopentyl beschrieben wird. In einer weiteren Ausführungsform sind Verbindungen der Formel I bevorzugt, in denen R6 und R7 unabhängig voneinander durch F, Cl, Br oder Methyl beschrieben werden, besonders bevorzugt sind Verbindungen, in denen R6 und R7 durch Cl beschrieben werden.
In einer weiteren Ausführungsform sind Verbindungen der Formel I bevorzugt, in denen R6 und R7 nicht durch Wasserstoff beschrieben werden.

Ganz besonders bevorzugt sind folgende Verbindungen der Formel 1:
(1H-Benzoimidazol-2-yl)-(2,6-dichlorphenyl)-methyl-amin,
(1H-Benzoimidazol-2-yl)-(2,6-dichlorphenyl)-ethyl-amin,(2,6-Dichlorphenyl)-(5-fluor-1H-benzoimidazol-2-yl)-methyl-amin,
(1H-Benzoimidazol-2-yl)-(2,6-dichlorphenyl)-isopropyl-aminAllyl-(1H-benzoimidazol-2-yl)-(2,6-dichlorphenyl)-amin oder
(1H-Benzoimidazol-2-yl)-cyclopentyl-(2,6-dichlorphenyl)-amin
sowie deren pharmazeutisch verträgliche Salze und Trifluoressigsäuresalze

Enthalten die Substituenten R1, R2, R3, R4, R5, R6 oder R7 ein oder mehrere Asymmetriezentren, so können diese unabhängig voneinander sowohl S als auch R konfiguriert sein. Die Verbindungen können als optische Isomere, als Diastereomere, als Racemate oder als Gemische derselben in allen Verhältnissen vorliegen.

Die vorliegende Erfindung umfasst alle tautomeren Formen der Verbindungen der Formel I.

Alkylreste können geradkettig oder verzweigt sein. Dies gilt auch, wenn sie Substituenten tragen oder als Substituenten anderer Reste auftreten, beispielsweise in Fluoralkylresten oder Alkoxyresten. Beispiele für Alkylreste sind Methyl, Ethyl, n-Propyl, Isopropyl (= 1-Methylethyl), n-Butyl, Isobutyl (= 2-Methylpropyl), sec-Butyl (= 1-Methylpropyl), tert-Butyl (= 1,1-Dimethylethyl). Bevorzugte Alkylreste sind Methyl, Ethyl, Isopropyl. In Alkylresten können ein oder mehrere, zum Beispiel 1, 2, 3, 4, 5, 6, 7, 8 oder 9, Wasserstoffatome durch Fluoratome substituiert sein. Beispiele für solche Fluoralkylreste sind Trifluormethyl, 2,2,2-Trifluorethyl, Pentafluorethyl, Heptafluorisopropyl. Substituierte Alkylreste können in beliebigen Positionen substituiert sein.

Alkenylreste können geradkettig oder verzweigt sein. Dies gilt auch, wenn sie Substituenten tragen beispielsweise in Fluoralkenylresten. Die Alkenylreste können in unterschiedlichen Positionen ungesättigt und auch mehrfach ungesättigt sein. Beispiele für Alkenylreste sind Ethenyl, n-Prop-1-enyl, n-Prop-2-enyl, Isoprop-1-enyl (= 1-Methylethenyl), n-But-1-enyl, n-But-2-enyl, n-But-3-enyl, n-But-1,3-dienyl, Isobut-1-enyl (= 2-Methylprop-1-enyl), Isobut-2-enyl (= 2-Methylprop-2-enyl), sec-But-1-enyl (= 1-Methylprop-1-enyl). Bevorzugte Alkenylreste sind Ethenyl, n-Prop-1-enyl, n-Prop-2-enyl, n-But-1-enyl, n-But-2-enyl. In Alkenylresten können ein oder mehrere, zum Beispiel 1, 2, 3, 4, 5, 6 oder 7, Wasserstoffatome durch Fluoratome substituiert sein. Substituierte Alkenylreste können in beliebigen Positionen substituiert sein.

Beispiele für Cycloalkylreste sind Cyclopropyl, Cyclobutyl, Cyclopentyl. In Cycloalkylresten können ein oder mehrere, zum Beispiel 1, 2, 3, 4, 5, 6, 7, 8 oder 9 Wasserstoffatome durch Fluoratome substituiert sein.

Beschrieben werden auch Methoden zur Herstellung der Verbindungen der Formel I. So lassen sich die durch Formel I beschriebenen Verbindungen in dem Fachmann bekannter Weise aus N-substituierten Cyanamiden der Formel II und den entsprechenden, käuflich in großer Zahl erhältlichen oder synthetisch leicht zugänglichen ortho-Phenylendiaminen der Formel III darstellen.

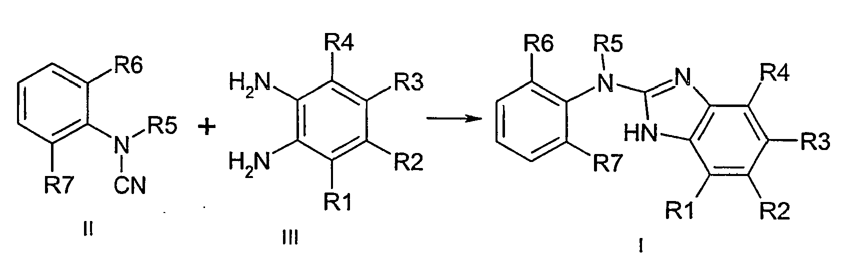

N-substituierte Cyanamide der Formel II lassen sich durch Umsetzung der korrespondierenden Cyanamide IV mit den Verbindungen R5-X erhalten. Dabei steht X für eine nukleophil substituierbare Gruppe - wie Chlor, Brom, lod, Alkylsulfonat oder Arylsulfonat.

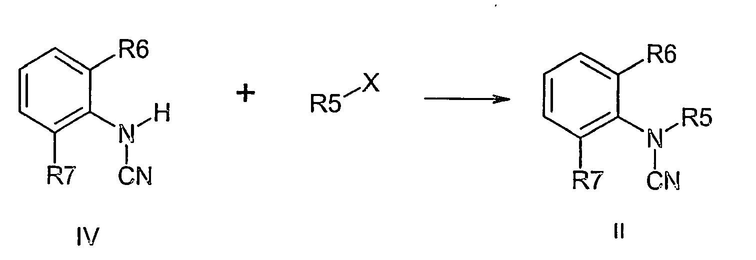

Die benötigten Cyanamide IV lassen sich anhand verschiedener literaturbekannter Methoden darstellen (Chem. Ber. 1908, 41, 524, J. Med. Chem. 1975, 18, 90-99, Org. Lett. 2000, 795).

Alternativ lassen sich Verbindungen der Formel I auch aus N-substituierten Anilinen der Formel V und Benzoimidazolderivaten der Formel VI in literaturbekannter Weise darstellen.

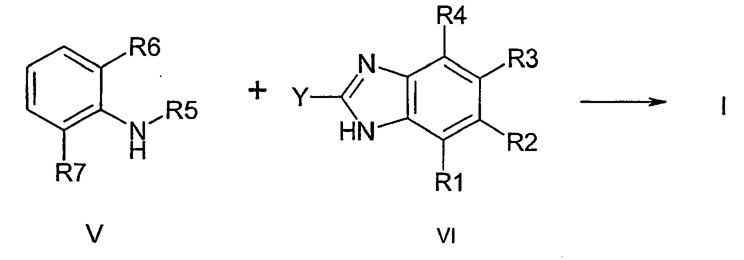

Dabei entspricht Y einer nukleophil substituierbaren Gruppe wie beispielsweise Chlor, Brom, lod, Alkylsulfonat wie Methansulfonat oder Trifluormethansulfonat oder Arylsulfonat wie Tosylat (analog Arch. Pharm. 1997, 330 (12) 372).

Weiterhin besteht auch die Möglichkeit, durch Umsetzung der 2-Anilinobenzoimidazole der Formel VII mit Verbindungen R5-X zu den Verbindungen der Formel I zu gelangen, wobei X wie oben beschrieben definiert ist. Verbindungen der Formel VII lassen sich nach literaturbekannten Methoden darstellen (WO 02 46169, Synthesis 1983, 861).

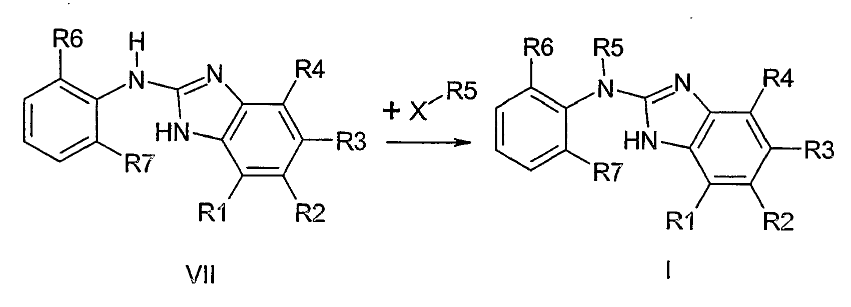

Als weiterer Zugang zu Verbindungen der Formel I bietet sich eine Synthesesequenz an, die von 2-Nitrophenyl-isothiocyanaten der Formel VIII ausgeht (J. Med. Chem. 1985, 28, 1925). Diese werden mit Aminen der Formel V zu Thioharnstoffen der Formel IX umgesetzt, die nach Reduktion, bevorzugt mit Wasserstoff in Gegenwart von Palladium/Kohle oder Platindioxid oder mit Zinn(IV)chlorid und Salzsäure, die Aminothioharnstoffe X liefern, die dann zu den Verbindungen der Formel I cyclisiert werden, bevorzugt in Gegenwart von Methyliodid (Mel) oder Dicyclohexylcarbodiimid (DCCI), das auch festphasengebunden sein kann.

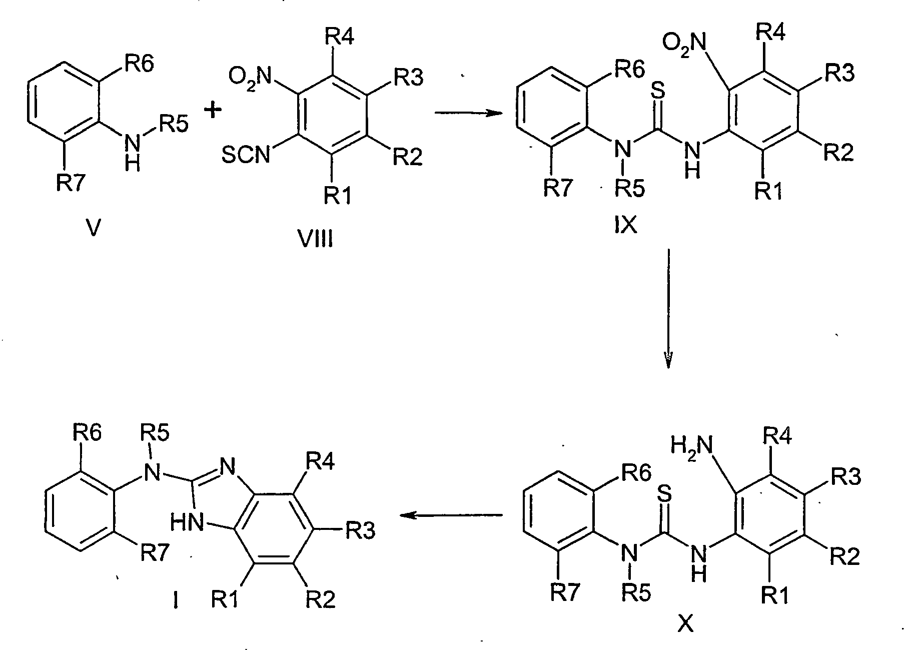

Alle Substituenten R1 bis R7, die in den Syntheseverfahren erwähnt sind, haben die Bedeutung wie für Verbindungen der Formel I definiert.

Bei der vorliegenden Erfindung konnte überraschend gezeigt werden, dass die beschriebenen Verbindungen potente Inhibitoren des Natrium/Wasserstoff-Austauschers (NHE) darstellen, insbesondere des NHE3.

Bekannte NHE3-Inhibitoren leiten sich beispielsweise von Verbindungen des Acylguanidin-Typs (EP 825 178), Norbornylamin-Typs (DE 199 60 204), 2-Guanidinochinazolin-Typs (WO 01 79 186, WO 02 20496) oder Benzamidin-Typs (WO 01 21582, WO 01 72 742) ab. Das ebenfalls als NHE3-Inhibitor beschriebene Squalamin (M. Donowitz et al., Am. J. Physiol. 276 (Cell Physiol. 45: C136 - C144) wirkt nicht unmittelbar wie die Verbindungen der Formel I, sondern erreicht seine maximale Wirkstärke erst nach einer Stunde.

In der deutschen Patentanmeldung DE10163239 werden NHE3-Inhibitoren vom Imidazolidin-Typ beschrieben. NHE3-Inhibitoren vom 2-Phenylaminobenzoimidazol-Typ sind kürzlich bekannt geworden (WO 02 46169). Alkylierungen bei Verbindungen dieser Art an der anilinischen Stickstoffposition waren bislang jedoch noch nicht beschrieben worden.
In der deutschen Patentanmeldung DE 10224892 werden NHE3-Inhibitoren vom Thiophen-Typ beschrieben. In der japanischen Patentschrift JP2869561 werden substituierte Benzoimidazole zur Inhibierung von Blutplättchen-Adhäsion beschrieben.

Überraschend wurde nun gefunden, dass Alkylierungen des Anilinstickstoffs nicht zum Verlust der NHE3-Aktivität führen. Das Wasserstoffatom am anilinischen Stickstoff scheint für die NHE3-Aktivität daher nicht essenziell zu sein. Gegenüber den oben beschriebenen 2-Phenylaminobenzoimidazolen zeichnen sich die hier beschriebenen Verbindungen durch eine höhere Lipophilie aus, wodurch das Hirn-Plasma-Verhältnis verbessert wird, was besonders für zentrale Indikationen von Bedeutung ist.

Das den hier beschriebenen Verbindungen ähnliche Clonidin ist als schwacher NHE-Inhibitor bekannt. Allerdings ist seine Wirkung auf den NHE3 der Ratte mit einer IC₅₀ von 620 µM äußerst moderat. Stattdessen weist es eine gewisse Selektivität für den NHE2 auf, an dem es eine IC₅₀ von 42 µM besitzt (J. Orlowski et al J., Biol. Chem. 268, 25536). Es ist daher eher als NHE2-Inhibitor zu bezeichnen. Neben der schwachen NHE-Wirkung besitzt das Clonidin eine hohe Affinität zum adrenergen alpha2-Rezeptor und Imidazolin I1-Rezeptor, wodurch eine starke blutdrucksenkende Wirkung vermittelt wird (Ernsberger et al, Eur. J. Pharmacol. 134, 1, 1987).

Verbindungen der Formel 1 zeichnen sich durch gesteigerte NHE3-Aktivität aus.

Der NHE3 wird im Körper verschiedener Spezies bevorzugt in der Galle, dem Darm und in der Niere gefunden (Larry Fliegel et al, Biochem. Cell. Biol. 76: 735 - 741, 1998), ließ sich aber auch im Gehirn nachweisen (E. Ma et al. Neuroscience 79: 591 - 603).

Aufgrund ihrer unerwarteten NHE-inhibitorischen Eigenschaften eignen sich die Verbindungen der Formel I zur Prävention und Behandlung von Krankheiten, die durch eine Aktivierung bzw. durch einen aktivierten NHE verursacht werden. Die Verwendung der erfindungsgemäßen Verbindungen betrifft die Prävention und die Behandlung akuter und chronischer Krankheiten in der Veterinär- und in der Humanmedizin.
So eignen sich die erfindungsgemäßen Inhibitoren des NHE zur Behandlung von Krankheiten, die durch Ischämie und / oder durch Reperfusion hervorgerufen werden. Die hier beschriebenen Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der Angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäß verwendeten Verbindungen der Formel I infolge Inhibition des zellulären Na⁺/H⁺-Austauschmechanismus als Arzneimittel zur Behandlung aller akuten oder chronischen durch lschämie ausgelösten Schäden oder dadurch primär oder sekundär induzierten Krankheiten verwendet werden. Dies betrifft ihre Verwendung als Arzneimittel für operative Eingriffe, z.B. bei Organ-Transplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeifien, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen.

Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind.

Darüber hinaus eignen sich die erfindungsgemäß verwendeten Verbindungen der Formel I ebenfalls zur Behandlung von Formen des Schocks, wie beispielweise des allergischen, cardiogenen, hypovolämischen und des bakteriellen Schocks.

Weiterhin induzieren die Verbindungen eine Verbesserung des Atemantriebes und werden deshalb zur Behandlung von Atmungszuständen bei folgenden klinischen Zuständen und Krankheiten herangezogen: Gestörter zentraler Atemantrieb (z. B. zentrale Schlafapnoen, plötzlicher Kindstod, postoperative Hypoxie), muskulärbedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.
Zusätzlich erhöhen die Verbindungen den Muskeltonus der oberen Atemwege, so dass das Schnarchen unterdrückt wird.

Eine Kombination eines NHE-Inhibitors mit einem Carboanhydrase-Hemmer (z. B. Acetazolamid), wobei letzterer eine metabolische Azidose herbeiführt und dadurch bereits die Atmungstätigkeit steigert, erweist sich als vorteilhaft durch verstärkte Wirkung und verminderten Wirkstoffeinsatz.

Außerdem eignen sich die hier beschriebenen Verbindungen als Medikamente zur Therapie und Prophylaxe von Erkrankungen und Störungen, die durch Übererregbarkeit des Zentralnervensystems ausgelöst werden, insbesondere zur Behandlung von Erkrankungen des epileptischen Formenkreises, zentral ausgelösten klonischen und tonischen Spasmen, psychischen Depressionszuständen, Angsterkrankungen und Psychosen. Dabei können die hier beschriebenen NHE-Inhibitoren allein angewandt werden oder in Kombination mit anderen antiepileptisch wirkenden Substanzen oder antipsychotischen Wirkstoffen, oder Carboanhydratasehemmern, beispielweise mit Acetazolamid, sowie mit weiteren Inhibitoren des NHE oder des Natrium-abhängigen Chlorid-Bicarbonat-Austauschers (NCBE).

Es hat sich gezeigt, dass die erfindungsgemäß verwendeten Verbindungen eine milde abführende Wirkung besitzen und demzufolge vorteilhaft als Abführmittel oder bei drohender Darmverstopfung verwendet werden können.

Außerdem können die erfindungsgemäßen Verbindungen vorteilhaft zur Prävention und Therapie von akuten und chronischen Erkrankungen des Darmtrakts verwendet werden, die durch ischämische Zustände im Intestinalbereich und / oder durch nachfolgende Reperfusion ausgelöst werden. Solche Komplikationen können beispielweise durch mangelnde Darmperistaltik hervorgerufen werden, wie sie z.B. häufig nach chirurgischen Eingriffen, bei Darmverstopfung oder stark verminderter Darmtätigkeit zu beobachten sind.

Weiterhin besteht die Möglichkeit, der Gallenstein-Bildung vorzubeugen.

Darüber hinaus zeichnen sich die erfindungsgemäß verwendeten Verbindungen der Formel I durch starke inhibierende Wirkung auf die Proliferation von Zellen, beispielsweise der Fibroblasten-Zellproliferation und der Proliferation der glatten Gefäßmuskelzellen, aus. Deshalb kommen die Verbindungen der Formel I als wertvolle Therapeutika für Krankheiten in Frage, bei denen die Zellproliferation eine primäre oder sekundäre Ursache darstellt, und können deshalb als Antiatherosklerotika, Mittel gegen diabetische Spätkomplikationen, Mittel gegen das chronische Nierenversagen, Krebserkrankungen, fibrotische Erkrankungen des Herzens, sowie Lungenfibrose, Leberfibrose oder Nierenfibrose, Organhypertrophien und -hyperplasien, beispielsweise des Herzens und der Prostata verwendet werden, und somit zur Prävention und Behandlung der Herinsuffizienz (congestive heart failure) oder bei Prostatahyperplasie bzw. Prostatahypertrophie benutzt werden.

Die erfindungsgemäßen Verbindungen sind wirkungsvolle Inhibitoren des zellulären Natrium-Protonen-Antiporters (Na/H-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäß verwendeten Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes und diabetischer Spätkomplikationen, proliferativer Erkrankungen usw.

Darüber hinaus sind die Verbindungen der Formel 1 für die präventive Therapie zur Verhinderung der Genese und zur Behandlung des Bluthochdrucks, beispielweise der essentiellen Hypertonie, geeignet, da sie die Rückresorption von NaCl im tubulären System der Niere vermindern oder vollständig inhibieren. Entsprechend sind sie auch hervorragend als Kombinations- und Formulierungspartner für Arzneimittel geeignet, die zur Bluthochdruckbehandlung verwendet werden. So können sie beispielsweise mit thiazid-artig wirkenden Diuretika, Loop-Diuretika, Aldosteron- und Pseudoaldosteron-Antagonisten, wie Hydrochlorothiazid, Indapamid, Polythiazid, Furosemid, Piretanid, Torasemid, Bumetanid, Amilorid, Triamteren kombiniert werden. Weiterhin können die NHE-Inhibitoren der vorliegenden Erfindung in Kombination mit ACE-Hemmern, wie beispielsweise Ramipril, Enalapril oder Captopril verwendet werden. Weitere günstige Kombinationspartner sind auch ß-Blocker.

Die beschriebenen NHE-Inhibitoren können ebenfalls in der Prävention und zur Behandlung thrombotischer Erkrankungen verwendet werden, da sie als NHE-Inhibitoren sowohl die Plättchenaggregation selbst inhibieren können und darüber hinaus die überschießende Freisetzung von Gerinnungsmediatoren, insbesondere des von Willebrand Faktors hemmen bzw. verhindern können. Deshalb sind die NHE-Inhibitoren der vorliegenden Erfindung kombinierbar mit weiteren antikoagulativen Wirkstoffen wie beispielsweise Acetylsalizylsäure, Thrombinantagonisten, Faktor Xa-Antagonisten, fibrinolytisch wirkenden Arzneistoffen, Faktor-Vlla-Antagonisten usw. Eine kombinierte Anwendung der vorliegenden NHE-Inhibitoren mit NCBE-Inhibitoren ist besonders günstig.

Es wurde außerdem gefunden, dass NHE-Inhibitoren eine günstige Beeinflussung der Serumlipoproteine zeigen. Es ist allgemein anerkannt, dass für die Entstehung arteriosklerotischer Gefäßveränderungen, insbesondere der koronaren Herzkrankheit, zu hohe Blutfettwerte, sogenannte Hyperlipoproteinämien, einen wesentlichen Risikofaktor darstellen. Für die Prophylaxe und die Regression von atherosklerotischen Veränderungen kommt daher der Senkung erhöhter SerumLipoproteine eine außerordentliche Bedeutung zu. Die erfindungsgemäß verwendeten Verbindungen können daher zur Prophylaxe und zur Regression von atherosklerotischen Veränderungen herangezogen werden, indem sie einen kausalen Risikofaktor ausschalten. Die erfindungsgemäßen Inhibitoren des NHE können in günstiger Weise auch mit anderen antiarteriosklerotischen Wirkstoffen kombiniert werden, wie einem Stoff aus der Klasse der Fibrate, einem Upregulator der LD2 Rezeptoraktivität, wie MD-700 und LY295427, oder einem Cholesterol- oder Gallensäure-Resorptionshemmer oder einem Antihyperchlesterolämikum aus der Klasse der Statine, wie beispielsweise Pravastatin, Lovastatin, Simvastatin. Mit diesem Schutz der Gefäße gegen das Syndrom der endothelialen Dysfunktion sind Verbindungen der Formel I wertvolle Arzneimittel zur Prävention und zur Behandlung koronarer Gefäßspasmen, peripherer Gefäßkrankheiten, wie claudicatio intermittens, der Atherogenese und der Atherosklerose, der linksventrikulären Hypertrophie und der dilatierten Kardiomyopathie, und thrombotischer Erkrankungen.

Die genannten Verbindungen können ebenfalls zur Behandlung von Krankheiten verwendet werden, die durch Protozoen verursacht werden und eignen sich insbesondere als Antimalariamittel.
Darüber hinaus eignen sich die Verbindungen zur Bekämpfung saugender Parasiten, wie Moskitos, Zecken, Flöhen und Pflanzenschädlingen.

Entsprechend ihrer protektiven Wirkungen sind die Verbindungen auch als Arzneimittel zur Gesunderhaltung und Lebensverlängerung geeignet.

Generell können die hier beschriebenen NHE-Inhibitoren in günstiger Weise mit anderen, den intrazellulären pH-regulierenden Verbindungen kombiniert werden,
wobei Inhibitoren der Enzymgruppe der Carboanhydratase, Inhibitoren der Bicarbonationen transportierenden Systeme wie des Natrium-Bicarbonat-Cotransporters oder des Natrium-abhängigen Chlorid-Bicarbonat-Austauschers, sowie andere NHF--inhibitoren, beispielweise mit inhibitorischer Wirkung auf andere NHE-Subtypen, als Kombinationspartner infrage kommen, weil durch sie die pharmakologisch relevanten pH-regulierenden Effekte der hier beschriebenen NHE-Inhibitoren verstärkt werden können.

Die genannten Verbindungen finden deshalb vorteilhaft Verwendung zur Herstellung eines Medikaments zur Prävention und Behandlung von Schlafapnoen und muskulär bedingter Atemstörungen; zur Herstellung eines Medikaments zur Prävention und Behandlung des Schnarchens; zur Herstellung eines Medikaments zur Blutdrucksenkung; zur Herstellung eines Medikaments mit abführender Wirkung zur Prävention und Behandlung intestinaler Verstopfungen; zur Herstellung eines Medikaments zur Prävention und Behandlung von Erkrankungen, die durch Ischämie und Reperfusion von zentralen und peripheren Organen ausgelöst werden wie das akute Nierenversagen, der Schlaganfall, endogene Schockzustände, Darmerkrankungen etc.; zur Herstellung eines Medikamentes zur Behandlung diabetischer Spätschäden und chronischer Nierenerkrankungen, insbesondere von allen Nierenentzündungen (Nephritiden), die mit einer vermehrten Protein/Albuminausscheidung verbunden sind; zur Herstellung eines Medikaments zur Behandlung von Hypercholesterinämie; zur Herstellung eines Medikaments zur Prävention der Atherogenese und der Atherosklerose; zur Herstellung eines Medikaments zur Prävention und Behandlung von Krankheiten, die durch erhöhte Cholesterinspiegel ausgelöst werden; zur Herstellung eines Medikaments zur Prävention und Behandlung von Krankheiten, die durch endotheliale Dysfunktion ausgelöst werden; zur Herstellung eines Medikaments zur Behandlung des Befalls durch Ektoparasiten; zur Herstellung eines Medikaments zur Behandlung der genannten Leiden in Kombinationen mit blutdrucksenkenden Stoffen, bevorzugt mit Angiotensin Converting Enzyme (ACE)-Hemmern, mit Diuretika, Aldosteron-Antagonisten oder Angiotensin-Rezeptorantagonisten. Eine Kombination eines NHE-Inhibitors der Formel I mit einem blutfettspiegelsenkenden Wirkstoff, bevorzugt mit einem HMG-CoA-Reduktaseinhibitor (z. B. Lovastatin oder Pravastatin), wobei letzterer eine hypolipidämische Wirkung herbeiführt und dadurch die hypolipidämischen Eigenschaften des NHE-Inhibitors der Formel 1 steigert, erweist sich ais günstige Kombination mit verstärkter Wirkung und vermindertem Wirkstoffeinsatz.

Beansprucht wird die Gabe von Natrium-Protonen-Austausch-Hemmern der Formel I als neuartige Arzneimittel zur Senkung erhöhter Blutfettspiegel, sowie die Kombination von Natrium-Protonen-Austausch-Hemmern mit blutdrucksenkenden und/oder hypolipidämisch wirkenden Arzneimitteln.

Die Erfindung betrifft auch Heilmittel für die humane, veterinäre oder phytoprotektive Anwendung enthaltend eine wirksame Menge einer Verbindung der Formel I und / oder eines pharmazeutisch verträglichen Salzes davon, ebenso wie Heilmittel für die humane, veterinäre oder phytoprotektive Anwendung enthaltend eine wirksame Menge einer Verbindung der Formel I und / oder eines pharmazeutisch verträglichen Salzes davon allein oder in Kombination mit einem oder mehreren anderen pharmakologischen Wirkstoffen oder Arzneimitteln.

Arzneimittel, die eine Verbindung der Formel I enthalten, können dabei zum Beispiel oral, parenteral, intravenös, rektal, topisch oder durch Inhalation appliziert werden,
wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinärals auch in der Humanmedizin und im Pflanzenschutz.

Die Hilfsstoffe, die für die gewünschte Arneimittelformulierung geeignet sind, sind dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wässrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die verwendeten aktiven Verbindungen, gewünschtenfalls mit den dafür üblichen Substanzen wie Lösungsvermittler, Emulgatoren oder weiteren Hilfsstoffen, in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.

Im Durchschnitt beträgt die tägliche Dosis einer Verbindung der Formel I bei einem etwa 75 kg schweren Patienten mindestens 0,001 mg/kg, vorzugsweise 0,1 mg/kg, bis höchstens 50 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation, können bis zu 200 mg/kg pro Tag notwendig werden.

Pharmazeutisch verträgliche Salze werden z. B. über folgende Säuren hergestellt: aus anorganischen Säuren wie Salzsäure, Schwefelsäure oder Phosphorsäure oder aus organischen Säuren wie Essigsäure, Zitronensäure, Weinsäure, Milchsäure, Malonsäure, Methansulfonsäure, Fumarsäure. Als Säureadditionssalze kommen dabei Salze aller pharmakologisch verträglichen Säuren in Frage, beispielsweise Halogenide, insbesondere Hydrochloride, Lactate, Sulfate, Citrate, Tartrate, Acetate, Phosphate, Methylsulfonate, p-Toluolsulfonate, Adipinate, Fumarate, Gluconate, Glutamate, Glycerolphosphate, Maleinate und Pamoate (diese Gruppe entspricht auch den physiologisch akzeptablen Anionen); aber auch Trifluoracetate.

### Versuchsbeschreibungen und Beispiele:

### Liste der verwendeten Abkürzungen:

- Rt: Retentionszeit
- LCMS: Liquid Chromatography Mass Spectroscopy
- MS: Mass Spectroscopy
- ES⁺: Electrospray, positive mode
- HPLC: High Performance Liquid Chromatography

### Allgemeines:

Die im Folgenden angegebenen Retentionszeiten (Rt) beziehen sich auf LCMS-Messungen mit den folgenden Parametern:

### Analytische Methode:

- stationäre Phase:: Merck Purospher 3µ 2 x 55 mm
- mobile Phase:: 95% H₂O (0,1 % HCOOH)→ 95% Acetonitril (0,1% HCOOH); 5 min→ 95% Acetonitril (0,1% HCOOH); 2 min → 95% H₂O (0,1 % HCOOH); 1 min; 0,45 ml/min.

Die präparative HPLC wurde unter folgenden Bedingungen durchgeführt:
- stationäre Phase:: Merck Purospher RP18 (10µM) 250 x 25 mm
- mobile Phase:: 90% H₂O (0,05% TFA)→ 90% Acetonitril; 40 min; 25 ml/min

### Beispiel 1: (1 H-Benzoimidazol-2-yl)-(2,6-dichlorphenyl)-ethyl-amin

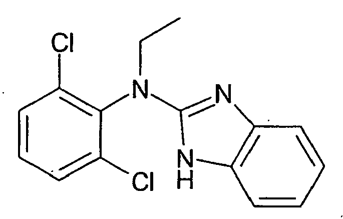

(1 H-Benzoimidazol-2-yl)-(2,6-dichlorphenyl)-amin (350 mg) wurde in Dimethylformamid (8 ml) gelöst. Zur entstandenen Lösung wurde Kaliumcarbonat (383 mg) gegeben und bei 0°C Ethyliodid (0,1 ml) unter Rühren zugetropft. Nach einer halben Stunde Rühren bei 0°C wurde bei Raumtemperatur weitergerührt. 2,5 Stunden später wurde das Reaktionsgemisch auf Eis-Wasser gegeben und die wässrige Phase dreimal mit Essigester extrahiert. Die vereinigten organischen Extrakte wurden mit gesättigter Natriumchlorid-Lösung gewaschen und anschließend über Magnesiumsulfat getrocknet. Nach Abziehen des Lösungsmittels im Vakuum wurde der Rückstand mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Essigester extrahiert. Nach Trocknen über Magnesiumsulfat wurde zur Trockne gebracht und auf Kieselgel mit Dichlormethan/Methanol 10/0,125 chromatographiert. Es wurden 12 mg der gewünschten Verbindung erhalten.
LCMS-Rt: 2,25 min
MS (ES⁺, M+H⁺): 306,09

### Beispiel 2: (1 H-Benzoimidazol-2-yl)-(2,6-dichlorphenyl)-methyl-amin-Hydrochlorid

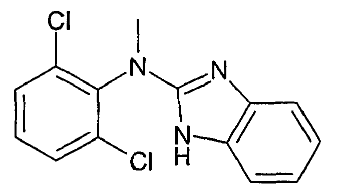

(1 H-Benzoimidazol-2-yl)-(2,6-dichlorphenyl)-amin (500 mg) wurde in Methanol (50 ml) gelöst. Zur entstandenen Lösung wurde Kaliumcarbonat (220 mg) gegeben, bei Raumtemperatur Methyliodid (248 mg) unter starkem Rühren zugetropft und anschließend auf Rückfluss erhitzt. Nach drei Tagen wurde das Reaktionsgemisch eingeengt, der Rückstand zwischen Essigester und Wasser verteilt und dann die Essigesterphase abgetrennt und getrocknet. Nach Einengen im Vakuum wurde der Rückstand mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Essigester extrahiert. Nach Trocknen über Magnesiumsulfat wurde zur Trockne gebracht und auf Kieselgel mit Essigester/Heptan 1/1 chromatographiert. Nach Vereinigen der Produkt enthaltenden Fraktionen und Abziehen des Lösungsmittels wurde der Rückstand mit wässriger Salzsäure aufgenommen und gefriergetrocknet. Es wurden 85 mg der gewünschten Verbindung erhalten.
LCMS-Rt: 2,03 min,
MS (ES⁺, M+H⁺): 292,05

### Alternativ lässt sich (1 H-Benzoimidazol-2-yl)-(2,6-dichlorphenyl)-methyl-amin-Hydrochlorid auch wie folgt gewinnen:

### a)(2,6-Dichlorphenyl)-methyl-cyanamid

(2,6-Dichlorphenyl)-cyanamid (1 g) wurde in trockenem Dimethylformamid (25 ml) gelöst, gepulvertes Kaliumcarbonat (739 mg) zugegeben und anschließend unter Rühren Methyliodid (1,52 g) zugetropft. Nach zwei Stunden Rühren bei Raumtemperatur wurde das Reaktionsgemisch eingeengt, der Rückstand mit Wasser aufgenommen, drei Mal mit Ether extrahiert und die vereinigten Etherextrakte über Magnesiumsulfat getrocknet. Nach Filtrieren wurde zur Trockne gebracht und der Rückstand mittels präparativer HPLC gereinigt. Es wurden 600 mg des gewünschten Produkts isoliert.
¹H-NMR (DMSO-d6/TMS, 400MHz): 7,67 (d, 10 Hz, 2H), 7,50 (t, 10 Hz, 1 H), 3,23 (s, 3H)

### b) (1 H-Benzoimidazol-2-yl)-(2,6-dichlorphenyl)-methyl-amin-Hydrochlorid

(2,6-Dichlorphenyl)-methyl-cyanamid (150 mg) und Phenylendiamin (81 mg) wurden in Hexafluor-2-propanol (1 ml) gelöst und in einem geschlossenen Gefäß 2 Tage auf 100 °C erhitzt. Nach einem weiteren Tag im offenen Gefäß bei 60 °C wurde das Lösungsmittel entfernt und der Rückstand mittels präparativer HPLC gereinigt. Die Produkt enthaltenden Fraktionen wurden vereinigt, das Acetonitril am Rotationsverdampfer abgezogen, der wässrige Rückstand mit Kaliumcarbonat neutralisiert und drei Mal mit Essigester extrahiert. Nach Trocknen über Magnesiumsulfat und Klären mit Kohle wurde zur Trockne gebracht. Der Rückstand wurde mit wässriger Salzsäure aufgenommen und gefriergetrocknet. Es wurden 5 mg der gewünschten Verbindung erhalten.

### Beispiel 3: (1 H-Benzoimidazol-2-yl)-(2,6-dichlorphenyl)-isopropyl-amin

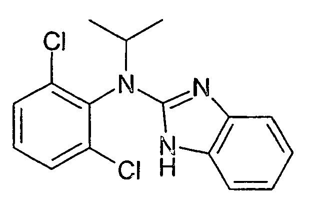

Analog zu Beispiel 1 wurde (1 H-Benzoimidazol-2-yl)-(2,6-dichlorphenyl)-amin (250 mg) in Dimethylformamid (3 ml) gelöst, mit Natriumhydrid (43 mg) deprotoniert und mit lsopropylbromid (111 mg) bei 100°C eine Stunde im geschlossenen Gefäß alkyliert. Nach Aufarbeitung und Chromatographie wurden 21 mg der gewünschten Verbindung erhalten.
LCMS-Rt: 2,30 min,
MS (ES⁺, M+H⁺): 320,16

### Beispiel 4: (2,6-Dichlorphenyl)-(5-fluor-1 H-benzoimidazol-2-yl)-methyl-amin

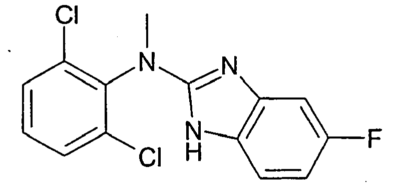

Analog zu Beispiel 1 wurde (2,6-Dichlorphenyl)-(5-fluor-1 H-benzoimidazol-2-yl)-amin (250 mg) in Dimethylformamid (2 ml) gelöst, mit Natriumhydrid (45 mg) deprotoniert und mit Methyliodid (120 mg) bei 100°C im geschlossenen Gefäß alkyliert.
Nach Aufarbeitung und Chromatographie wurden 19 mg der gewünschten Verbindung erhalten.
LCMS-RT: 2,16 min,
MS (ES⁺, M+H⁺): 310,12

### Beispiel 5: Allyl-(1H-benzoimidazol-2-yl)-(2,6-dichlorphenyl)-amin

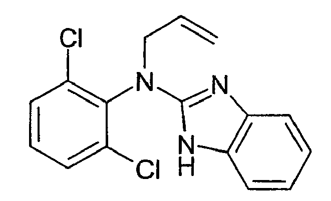

Unter Argon wurde in einem Mikrowellen-tauglichen Gefäß (1 H-Benzoimidazol-2-yl)-(2,6-dichlorphenyl)-amin (250 mg) in Dimethylformamid (2 ml) gelöst und mit Natriumhydrid (43 mg) versetzt. Nach einer Stunde Rühren bei Raumtemperatur wurde Cyclopropylbromid (109 mg) zugetropft und eine weitere halbe Stunde gerührt. Anschließend wurde das Gefäß zunächst 10 min in die Mikrowellenapparatur gestellt (80°C, 100W) und dann noch einmal 60 min (110°C, 100W). Nach weiterer Zugabe von Cyclopropylbromid (55 mg) wurde nochmals 60 min in die Mikrowelle gestellt (110°C, 100W).
Nach Aufarbeitung und Kieselgel-Chromatographie (siehe Beispiel 1) wurden 13 mg der gewünschten Verbindung erhalten.
LCMS-Rt: 2,28 min,
MS (ES⁺, M+H⁺): 318,20

### Beispiel 6: (1 H-Benzoimidazol-2-yl)-cyclopentyl-(2,6-dichlorphenyl)-amin

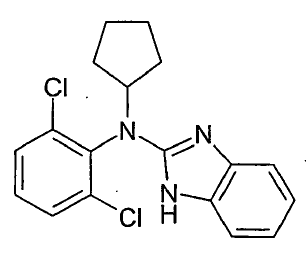

Analog zu Beispiel 1 wurde bei Raumtemperatur (1 H-Benzoimidazol-2-yl)-(2,6-dichlorphenyl)-amin (250 mg) in Dimethylformamid (2 ml) gelöst, mit Natriumhydrid (48 mg) deprotoniert und mit Cyclopentylbromid (136,7 mg) bei 100°C 8 h im geschlossenen Gefäß alkyliert.
Nach Aufarbeitung und Chromatographie wurden 38 mg der gewünschten Verbindung erhalten.
LCMS-Rt: 2,60 min,
MS (ES⁺, M+H⁺): 346,22

### Pharmakologische Daten:

### Testbeschreibung:

In diesem Test wurde die Erholung des intrazellulären pHs (pHᵢ) nach einer Ansäuerung ermittelt, die bei funktionsfähigem NHE3 auch unter bicarbonatfreien Bedingungen einsetzt. Dazu wurde der pHᵢ mit dem pH-sensitiven Fluoreszenzfarbstoff BCECF (Calbiochem, eingesetzt wird die Vorstufe BCECF-AM) bestimmt. Die Zellen (Fibroblasten, LAP1 Zellen) wurden zunächst mit BCECF beladen. Die BCECF-Fluoreszenz wurde in einem "Ratio Fluorescence Spectrometer" (Photon Technology International, South Brunswick, N.J., USA) bei Anregungswellenlängen von 505 und 440 nm und einer Emissionswellenlänge von 535 nm bestimmt und mittels Kalibrierungskurven in den pHᵢ umgerechnet. Die Zellen wurden bereits bei der BCECF-Beladung in NH₄Cl-Puffer (pH 7,4) inkubiert (NH₄Cl-Puffer: 115 mM NaCl, 20 mM NH₄Cl, 5 mM KCI, 1 mM CaCl₂, 1 mM MgSO₄, 20 mM Hepes, 5 mM Glucose, 1 mg/ml BSA; mit 1 M NaOH wird ein pH von 7,4 eingestellt).

Die intrazelluläre Ansäuerung wurde durch Zugabe von 975 µl eines NH₄Cl -freien Puffers (s. u.) zu 25 µl Aliquots der in NH₄Cl -Puffer inkubierten Zellen induziert. Die nachfolgende Geschwindigkeit der pH-Erholung wurde drei Minuten registriert. Für die Berechnung der inhibitorischen Potenz der getesteten Substanzen wurden die Zellen zunächst in Puffern untersucht, bei denen eine vollständige bzw. überhaupt keine pH-Erholung stattfand. Zur vollständigen pH-Erholung (100%) wurden die Zellen in Na⁺-haltigem Puffer inkubiert (133,8 mM NaCl, 4,7 mM KCI, 1,25 mM CaCl₂, 1,25 mM MgCl₂, 0,97 mM Na₂HPO₄, 0,23 mM NaH₂PO₄, 5 mM Hepes, 5 mM Glucose, mit 1 M NaOH wird ein pH von 7,0 eingestellt). Für die Bestimmung des 0%-Wertes wurden die Zellen in einem Na⁺-freien Puffer inkubiert (133,8 mM Cholinchlorid, 4,7 mM KCl, 1,25 mM CaCl₂, 1,25 mM MgCl₂, 0,97 mM K₂HPO₄, 0,23 mM KH₂PO₄, 5 mM Hepes, 5 mM Glucose, mit 1 M KOH wird ein pH von 7,0 eingestellt). Die zu testenden Substanzen wurden in dem Na⁺-haltigem Puffer angesetzt. Die Erholung des intrazellulären pH's bei jeder getesteten Konzentration einer Substanz wurde in Prozent der maximalen Erholung ausgedrückt. Aus den Prozentwerten der pH-Erholung wurde mittels des Programms Sigma-Plot der IC₅₀-Wert der jeweiligen Substanz für die einzelnen NHE-Subtypen berechnet.

### Ergebnisse:

| Beispiel | IC₅₀ [µM], (rNHE3) |
|---|---|
| 1 | 0,57 |
| 2 | 0,53 |
| 3 | 9,3 |
| 4 | 3,3 |
| 5 | 1,5 |
| 6 | 8,3 |

## Patentansprüche

1. Verbindungen der Formel I, 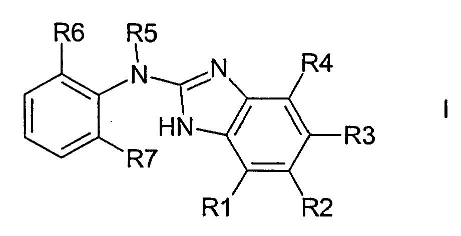 worin bedeuten:
R1 und R4 unabhängig voneinander H, F, Cl, Br, Alkyl mit 1, 2, 3 oder 4 C-Atomen oder Alkoxy mit 1, 2, 3 oder 4 C-Atomen,
wobei die Alkyl- und Alkoxygruppen unsubstituiert sind oder unabhängig voneinander mit 1, 2, 3, 4, 5, 6, 7, 8 oder 9 F-Atomen substituiert sind;
R2 und R3 unabhängig voneinander H, F, Alkyl mit 1, 2 oder 3 C-Atomen, Alkoxy mit 1, 2 oder 3 C-Atomen oder OH,
wobei die Alkyl- und Alkoxygruppen unsubstituiert sind oder unabhängig voneinander mit 1, 2, 3, 4, 5, 6 oder 7 F-Atomen substituiert sind;
R5 Alkyl mit 1, 2, 3 oder 4 C-Atomen, Alkenyl mit 2, 3 oder 4 C-Atomen oder Cycloalkyl mit 3, 4 oder 5 C-Atomen,
wobei die Alkyl-, Alkenyl-, und Cycloalkylgruppen unsubstituiert sind oder mit 1, 2, 3, 4, 5, 6, 7, 8 oder 9 F-Atomen substituiert sind;
R6 und R7 unabhängig voneinander H, F, Cl, Br, Alkyl mit 1, 2, 3 oder 4 C-Atomen oder Alkoxy mit 1, 2 oder 3 C-Atomen,
wobei die Alkyl- und Alkoxygruppen unsubstituiert sind oder unabhängig voneinander mit 1, 2, 3, 4, 5, 6, 7, 8 oder 9 F-Atomen substituiert sind,
und wenigstens einer der beiden Reste, R6 oder R7, nicht Wasserstoff entspricht;
sowie deren pharmazeutisch verträgliche Salze und Trifluoressigsäure-salze.

2. Verbindungen der Formel I nach Anspruch 1,
worin bedeuten:
R1 und R4 unabhängig voneinander H, F, CI, Br, Alkyl mit 1, 2, 3 oder 4 C-Atomen oder Alkoxy mit 1, 2, 3 oder 4 C-Atomen,
wobei die Alkyl- und Alkoxygruppen unsubstituiert sind oder unabhängig voneinander mit 1, 2, 3, 4, 5, 6, 7, 8 oder 9 F-Atomen substituiert sind;
R2 und R3 unabhängig voneinander H, F, Alkyl mit 1, 2 oder 3 C-Atomen, Alkoxy mit 1, 2 oder 3 C-Atomen oder OH,
wobei die Alkyl- und Alkoxygruppen unsubstituiert sind oder unabhängig voneinander mit 1, 2, 3, 4, 5, 6 oder 7 F-Atomen substituiert sind;
R5 Alkyl mit 1, 2, 3 oder 4 C-Atomen, Alkenyl mit 2, 3 oder 4 C-Atomen oder Cycloalkyl mit 3, 4 oder 5 C-Atomen,
wobei die Alkyl-, Alkenyl-, und Cycloalkylgruppen unsubstituiert sind oder mit 1, 2, 3, 4, 5, 6, 7, 8 oder 9 F-Atomen substituiert sind;
R6 und R7 unabhängig voneinander F, Cl, Br, Alkyl mit 1, 2 oder 3 C-Atomen oder Alkoxy mit 1, 2 oder 3 C-Atomen,
wobei die Alkyl- und Alkoxygruppen unsubstituiert sind oder unabhängig voneinander mit 1, 2, 3, 4, 5, 6 oder 7 F-Atomen substituiert sind;
und wenigstens einer der beiden Reste, R6 oder R7, nicht Wasserstoff entspricht;
sowie deren pharmazeutisch verträgliche Salze und Trifluoressigsäure-salze.

3. Verbindungen der Formel I nach Anspruch 2, **dadurch gekennzeichnet, dass** sie ausgewählt sind aus der Gruppe:
(1H-Benzoimidazol-2-yl)-(2,6-dichlorphenyl)-methyl-amin,
(1 H-Benzoimidazol-2-yl)-(2,6-dichlorphenyl)-ethyl-amin, (2,6-Dichlorphenyl)-(5-fluor-1H-benzoimidazol-2-yl)-methyl-amin,
(1 H-Benzoimidazol-2-yl)-(2,6-dichlorphenyl)-isopropyl-amin, Allyl-(1 H-benzoimidazol-2-yl)-(2,6-dichlorphenyl)-amin,
(1 H-Benzoimidazol-2-yl)-cyclopentyl-(2,6-dichlorphenyl)-amin,sowie deren pharmazeutisch verträgliche Salze und Trifluoressigsäuresalze.

4. Verbindungen der Formel I und deren pharmazeutisch verträgliche Salze nach einem oder mehreren der Ansprüche 1 bis 3 zur Verwendung als Medikament.

5. Verwendung einer Verbindung der Formel I und/oder deren pharmazeutisch verträglicher Salze nach einem oder mehreren der Ansprüche 1 bis 3 zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

6. Verwendung einer Verbindung der Formel I und/oder deren pharmazeutisch verträgliche Salze nach einem oder mehreren der Ansprüche der Ansprüche 1 bis 3 in Kombination mit anderen Arzneimitteln oder Wirkstoffen zur Herstellung eines Medikaments zur Behandlung oder Prophylaxe von Störungen des Atemantriebs, von Atemstörungen, Schlaf-bedingten Atemstörungen, Schlafapnoen, von Schnarchen, von akuten und chronischen Nierenerkrankungen, des akuten Nierenversagens und des chronischen Nierenversagens, von Störungen der Darmfunktion, des Bluthochdrucks, der essentiellen Hypertonie, von Erkrankungen des Zentralnervensystems, von Erkrankungen, die durch ZNS-Übererregbarkeit resultieren, Epilepsie und zentral ausgelöste Krämpfe oder von Angstzuständen, Depressionen und Psychosen, von ischämischen Zuständen des peripheren oder zentralen Nervensystems oder des Schlaganfalls, von akuten und chronischen Schäden und Erkrankungen peripherer Organe oder Gliedmaßen, die durch Ischämie- oder durch Reperfusionsereignisse verursacht werden, von Atherosklerose, von Störungen des Fettstoffwechsels, von Thrombosen, von Störungen der Gallenfunktion, von Befall durch Ektoparasiten, von Erkrankungen infolge endothelialer Dysfunktion, von Protozoen-Erkrankungen, der Malaria, zur Konservierung und Lagerung von Transplantaten für chirurgische Maßnahmen, für den Einsatz bei chirurgischen Operationen und Organtransplantationen oder zur Behandlung von Schockzuständen oder der Zuckerkrankheit und diabetischer Spätschäden oder von Krankheiten, bei denen die Zellproliferation eine primäre oder sekundäre Ursache darstellt und zur Gesunderhaltung und Lebensverlängerung.

7. Verwendung einer Verbindung der Formel I und/oder deren pharmazeutisch verträglicher Salze nach einem oder mehreren der Ansprüche 1 bis 3 allein oder in Kombination mit anderen Arzneimitteln oder Wirkstoffen zur Herstellung eines Medikaments zur Behandlung oder Prophylaxe von Störungen des Atemantriebs und/oder von Schlaf-bedingten Atemstörungen wie Schlafapnoen.

8. Verwendung einer Verbindung der Formel I und/oder deren pharmazeutisch verträglicher Salze nach einem oder mehreren der Ansprüche 1 bis 3 allein oder in Kombination mit anderen Arzneimitteln oder Wirkstoffen zur Herstellung eines Medikaments zur Behandlung oder Prophylaxe des Schnarchens.

9. Verwendung einer Verbindung der Formel I und/oder deren pharmazeutisch verträglicher Salze nach einem oder mehreren der Ansprüche 1 bis 3 allein oder in Kombination mit anderen Arzneimitteln oder Wirkstoffen zur Herstellung eines Medikaments zur Behandlung oder zur Prophylaxe von akuten oder chronischen Nierenerkrankungen, des akuten Nierenversagens oder des chronischen Nierenversagens.

10. Verwendung einer Verbindung der Formel und/oder deren pharmazeutisch verträglicher Salze nach einem oder mehreren der Ansprüche 1 bis 3 allein oder in Kombination mit anderen Arzneimitteln oder Wirkstoffen zur Herstellung eines Medikaments zur Behandlung oder Prophylaxe von Störungen der Darmfunktion.

11. Heilmittel für die humane, veterinäre oder phytoprotektive Anwendung, enthaltend eine wirksame Menge einer Verbindung der Formel I und/oder eines pharmazeutisch verträglichen Salzes davon nach einem oder mehreren der Ansprüche 1 bis 3.

12. Heilmittel für die humane, veterinäre oder phytoprotektive Anwendung, enthaltend eine wirksame Menge einer Verbindung der Formel I und/oder eines pharmazeutisch verträglichen Salzes davon nach einem oder mehreren der Ansprüche 1 bis 3 in Kombination mit einem oder mehreren anderen pharmakologischen Wirkstoffen oder Arzneimitteln.

## Claims

1. A compound of the formula I 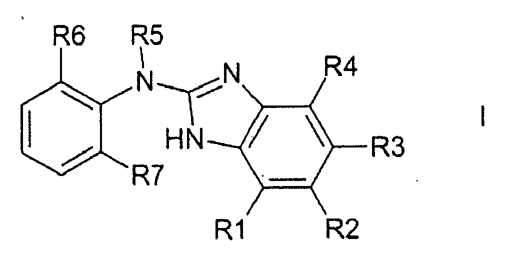 in which the meanings are:
R1 and R4 independently of one another H, F, Cl, Br, alkyl having 1, 2, 3 or 4 C atoms or alkoxy having 1, 2, 3 or 4 C atoms,
where the alkyl and alkoxy groups are unsubstituted or are substituted independently of one another by 1, 2, 3, 4, 5, 6, 7, 8 or 9 F atoms;
R2 and R3 independently of one another H, F, alkyl having 1, 2 or 3 C atoms, alkoxy having 1, 2 or 3 C atoms or OH,
where the alkyl and alkoxy groups are unsubstituted or are substituted independently of one another by 1, 2, 3, 4, 5, 6 or 7 F atoms;
R5 alkyl having 1, 2, 3 or 4 C atoms, alkenyl having 2, 3 or 4 C atoms or cycloalkyl having 3, 4 or 5 C atoms,
where the alkyl, akenyl and cycloalkyl groups are unsubstituted or are substituted by 1, 2, 3, 4, 5, 6, 7, 8 or 9 F atoms;
R6 and R7 independently of one another H, F, Cl, Br, alkyl having 1, 2, 3 or 4 C atoms or alkoxy having 1, 2 or 3 C atoms,
where the alkyl and alkoxy groups are unsubstituted or are substituted independently of one another by 1, 2, 3, 4, 5, 6, 7, 8 or 9 F atoms,
and at least one of the two radicals R6 or R7 does not correspond to hydrogen;
and the pharmaceutically acceptable salts and trifluoroacetic acid salts thereof.

2. A compound of the formula I as claimed in claim 1,
in which the meanings are:
R1 and R4 independently of one another H, F, CI, Br, alkyl having 1, 2, 3 or 4 C atoms or alkoxy having 1, 2, 3 or 4 C atoms,
where the alkyl and alkoxy groups are unsubstituted or are substituted independently of one another by 1, 2, 3, 4, 5, 6, 7, 8 or 9 F atoms;
R2 and R3 independently of one another H, F, alkyl having 1, 2 or 3 C atoms, alkoxy having 1, 2 or 3 C atoms or OH,
where the alkyl and alkoxy groups are unsubstituted or are substituted independently of one another by 1, 2, 3, 4, 5, 6 or 7 F atoms;
R5 alkyl having 1, 2, 3 or 4 C atoms, alkenyl having 2, 3 or 4 C atoms or cycloalkyl having 3, 4 or 5 C atoms,
where the alkyl, alkenyl and cycloalkyl groups are unsubstituted or are substituted by 1, 2, 3, 4, 5, 6, 7, 8 or 9 F atoms;
R6 and R7 independently of one another F, Cl, Br, alkyl having 1, 2 or 3 C atoms or alkoxy having 1, 2 or 3 C atoms,
where the alkyl and alkoxy groups are unsubstituted or are substituted independently of one another by 1, 2, 3, 4, 5, 6 or 7 F atoms;
and at least one of the two radicals R6 or R7 does not correspond to hydrogen;
and the pharmaceutically acceptable salts and trifluoroacetic acid salts thereof.

3. A compound of the formula I as claimed in claim 2, which is selected from the group:
(1 H-benzoimidazol-2-yl)(2,6-dichlorophenyl)methylamine
(1 H-benzoimidazol-2-yl)(2,6-dichlorophenyl)ethylamine (2,6-dichlorophenyl)(5-fluoro-1 H-benzoimidazol-2-yl)methylamine
(1 H-benzoimidazol-2-yl)(2,6-dichlorophenyl)isopropylamine allyl(1 H-benzoimidazol-2-yl)(2,6-dichlorophenyl)amine
(1 H-benzoimidazol-2-yl)cyclopentyl(2,6-dichlorophenyl)amine
and the pharmaceutically acceptable salts and trifluoroacetic acid salts thereof.

4. A compound of the formula I and the pharmaceutically acceptable salts thereof as claimed in one or more of claims 1 to 3 for use as medicament.

5. The use of a compound of the formula I and/or the pharmaceutically acceptable salts thereof as claimed in one or more 1 to 3 for producing a medicament for the treatment or prophylaxis of disorders of respiratory drive, of respiratory disorders, sleep-related respiratory disorders, sleep apneas, of snoring, of acute and chronic renal disorders, of acute renal failure and of chronic renal failure, of disorders of intestinal function, of high blood pressure, of essential hypertension, of disorders of the central nervous system, of disorders resulting from CNS overexcitability, epilepsy and centrally induced convulsions or of anxiety states, depressions and psychoses, of ischemic states of the peripheral or central nervous system or of stroke, of acute and chronic damage to and disorders of peripheral organs and limbs caused by ischemic or by reperfusion events, of atherosclerosis, of disorders of lipid metabolism, of thromboses, of disorders of biliary function, of infestation by ectoparasites, of disorders resulting from endothelial dysfunction, of protozoal disorders, of malaria, for the preservation and storage of transplants for surgical procedures, for use in surgical operations and organ transplantations or for the treatment of states of shock or of diabetes and late damage from diabetes or of diseases in which cellular proliferation represents a primary or secondary cause, and for maintaining health and prolonging life.

6. The use of a compound of the formula I and/or the pharmaceutically acceptable salts thereof as claimed in one or more of claims 1 to 3 in combination with other drugs or active ingredients for producing a medicament for the treatment or prophylaxis of disorders of respiratory drive, of respiratory disorders, sleep-related respiratory disorders, sleep apneas, of snoring, of acute and chronic renal disorders, of acute renal failure and of chronic renal failure, of disorders of intestinal function, of high blood pressure, of essential hypertension, of disorders of the central nervous system, of disorders resulting from CNS overexcitability, epilepsy and centrally induced convulsions or of anxiety states, depressions and psychoses, of ischemic states of the peripheral or central nervous system or of stroke, of acute and chronic damage to and disorders of peripheral organs and limbs caused by ischemic or by reperfusion events, of atherosclerosis, of disorders of lipid metabolism, of thromboses, of disorders of biliary function, of infestation by ectoparasites, of disorders resulting from endothelial dysfunction, of protozoal disorders, of malaria, for the preservation and storage of transplants for surgical procedures, for use in surgical operations and organ transplantations or for the treatment of states of shock or of diabetes and late damage from diabetes or of diseases in which cellular proliferation represents a primary or secondary cause, and for maintaining health and prolonging life.

7. The use of a compound of the formula I and/or of pharmaceutically acceptable salts thereof as claimed in one or more of claims 1 to 3, alone or in combination with other drugs or active ingredients for producing a medicament for the treatment or prophylaxis of disorders of respiratory drive and/or of sleep-related respiratory disorders such as sleep apneas.

8. The use of a compound of the formula I and/or of pharmaceutically acceptable salts thereof as claimed in one or more of claims 1 to 3, alone or in combination with other drugs or active ingredients for producing a medicament for the treatment or prophylaxis of snoring.

9. The use of a compound of the formula I and/or of pharmaceutically acceptable salts thereof as claimed in one or more of claims 1 to 3, alone or in combination with other drugs or active ingredients for producing a medicament for the treatment or prophylaxis of acute or chronic renal disorders, of acute renal failure or of chronic renal failure.

10. The use of a compound of the formula I and/or of pharmaceutically acceptable salts thereof as claimed in one or more of claims 1 to 3, alone or in combination with other drugs or active ingredients for producing a medicament for the treatment or prophylaxis of disorders of intestinal function.

11. A curative composition for human, veterinary or phytoprotective use comprising an effective amount of a compound of the formula I and/or of a pharmaceutically acceptable salt thereof as claimed in one or more of claims 1 to 3.

12. A curative composition for human, veterinary or phytoprotective use comprising an effective amount of a compound of the formula I and/or of a pharmaceutically acceptable salt thereof as claimed in one or more of claims 1 to 3, in combination with one or more other pharmacological active ingredients or drugs.

## Revendications

1. Composés de formule I 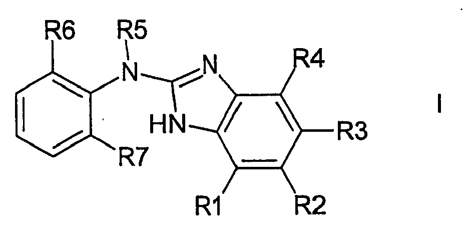 dans laquelle
R1 et R4 représentent, indépendamment l'un de l'autre, H, F, Cl, Br, alkyle comprenant 1, 2, 3 ou 4 atomes de carbone ou alcoxy comprenant 1, 2, 3 ou 4 atomes de carbone ;
où les groupes alkyle et alcoxy sont non substitués ou substitués, indépendamment l'un de l'autre, par 1, 2, 3, 4, 5, 6, 7, 8 ou 9 atomes de fluor ;
R2 et R3 représentent, indépendamment l'un de l'autre, H, F, alkyle comprenant 1, 2 ou 3 atomes de carbone, alcoxy comprenant 1, 2
ou 3 atomes de carbone ou OH ;
où les groupes alkyle et alcoxy sont non substitués ou substitués, indépendamment l'un de l'autre, par 1, 2, 3, 4, 5, 6 ou 7 atomes de fluor ;
R5 représente alkyle comprenant 1, 2, 3 ou 4 atomes de carbone, alcényle comprenant 2, 3 ou 4 atomes de carbone ou cycloalkyle comprenant 3, 4 ou 5 atomes de carbone ;
où les groupes alkyle, alcényle et cycloalkyle sont non substitués ou substitués par 1, 2, 3, 4, 5, 6, 7, 8 ou 9 atomes de fluor ;
R6 et R7 représentent, indépendamment l'un de l'autre, H, F, Cl, Br, alkyle comprenant 1, 2, 3 ou 4 atomes de carbone ou alcoxy comprenant 1, 2 ou 3 atomes de carbone ;
où les groupes alkyle et alcoxy sont non substitués ou substitués, indépendamment l'un de l'autre, par 1, 2, 3, 4, 5, 6, 7, 8 ou 9 atomes de fluor,
et au moins un des deux radicaux R6 ou R7 ne correspond pas à de l'hydrogène ;
ainsi que leurs sels pharmaceutiquement acceptables et les sels de l'acide trifluoroacétique.

2. Composés de formule I selon la revendication 1, dans laquelle :
R1 et R4 représentent, indépendamment l'un de l'autre, H, F, CI, Br, alkyle comprenant 1, 2, 3, ou 4 atomes de carbone ou alcoxy comprenant 1, 2, 3 ou 4 atomes de carbone ;
où les groupes alkyle et alcoxy sont non substitués ou substitués, indépendamment l'un de l'autre, par 1, 2, 3, 4, 5, 6, 7, 8 ou 9 atomes de fluor ;
R2 et R3 représentent, indépendamment l'un de l'autre, H, F, alkyle comprenant 1, 2 ou 3 atomes de carbone, alcoxy comprenant 1, 2
ou 3 atomes de carbone ou OH ;
où les groupes alkyle et alcoxy sont non substitués ou substitués, indépendamment l'un de l'autre, par 1, 2, 3, 4, 5, 6 ou 7 atomes de fluor ;
R5 représente alkyle comprenant 1, 2, 3 ou 4 atomes de carbone, alcényle comprenant 2, 3 ou 4 atomes de carbone ou cycloalkyle comprenant 3, 4 ou 5 atomes de carbone ;
où les groupes alkyle, alcényle et cycloalkyle sont non substitués
ou substitués par 1, 2, 3, 4, 5, 6, 7, 8 ou 9 atomes de fluor ;
R6 et R7 représentent, indépendamment l'un de l'autre, F, CI, Br, alkyle comprenant 1, 2 ou 3 atomes de carbone ou alcoxy comprenant 1, 2 ou 3 atomes de carbone ;
où les groupes alkyle et alcoxy sont non substitués ou substitués, indépendamment l'un de l'autre, par 1, 2, 3, 4, 5, 6 ou 7 atomes de fluor ;
et au moins un des deux radicaux R6 ou R7 ne correspond pas à de l'hydrogène ;
ainsi que leurs sels pharmaceutiquement acceptables et les sels de l'acide trifluoroacétique.

3. Composés de formule I selon la revendication 2, **caractérisés en ce qu'**ils sont choisis dans le groupe formé par :
la (1 H-benzoimidazol-2-yl)-(2,6-dichlorophényl)-méthylamine,
la (1 H-benzoimidazol-2-yl)-(2,6-dichlorophényl)-éthylamine,
la (2,6-dichlorophényl)-(5-fluoro-1 H-benzoimidazol-2-yl)-méthylamine,
la (1 H-benzoimidazol-2-yl)-(2,6-dichlorophényl)-isopropylamine, l'allyl-(1 H-benzoimidazol-2-yl)-(2,6-dichlorophényl)-amine,
la (1 H-benzoimidazol-2-yl)-cyclopentyl-(2,6-dichlorophényl)-amine,
ainsi que leurs sels pharmaceutiquement acceptables et les sels de l'acide trifluoroacétique.

4. Composés de formule I et leurs sels pharmaceutiquement acceptables selon l'une ou plusieurs des revendications 1 à 3 pour une utilisation comme médicament.

5. Utilisation d'un composé de formule I et/ou de ses sels pharmaceutiquement acceptables selon l'une ou plusieurs des revendications 1 à 3 pour la préparation d'un médicament destiné au traitement ou à la prophylaxie de troubles de l'effort respiratoire, de troubles respiratoires, de troubles respiratoires provoqués par le sommeil, des apnées du sommeil, du ronflement, de maladies aiguës et chroniques des reins, de la défaillance rénale aiguë et de la défaillance rénale chronique, de troubles de la fonction intestinale, de l'hypertension, de l'hypertonie essentielle, de maladies du système nerveux central, de maladies qui résultent d'une surexcitabilité du SNC, de l'épilepsie et de crampes déclenchées de manière centrale ou d'états d'anxiété, de dépressions et de psychoses, d'états ischémiques du système nerveux périphérique ou central ou d'une attaque, de dommages et de maladies aigus et chroniques d'organes périphériques ou de membres, qui sont provoqués par des événements d'ischémie ou de reperfusion, de l'athérosclérose, de troubles du métabolisme des graisses, de thromboses, de troubles de la fonction biliaire, d'une attaque par des ectoparasites, de maladies suite à un dysfonctionnement endothélial, de maladies dues à des protozoaires, de la malaria, à la conservation et à l'entreposage de greffes destinées à des interventions chirurgicales, à une utilisation lors d'opérations chirurgicales et de transplantations d'organes ou au traitement d'états de choc ou du diabète et des dommages diabétiques tardifs ou de maladies dans lesquelles la prolifération cellulaire représente une cause primaire ou secondaire et aux soins de santé et à la prolongation de la vie.

6. Utilisation d'un composé de formule I et/ou de ses sels pharmaceutiquement acceptables selon l'une ou plusieurs des revendications 1 à 3 en combinaison avec d'autres médicaments ou substances actives pour la préparation d'un médicament destiné au traitement ou à la prophylaxie de troubles de l'effort respiratoire, de troubles respiratoires, de troubles respiratoires provoqués par le sommeil, des apnées du sommeil, du ronflement, de maladies aiguës et chroniques des reins, de la défaillance rénale aiguë et de la défaillance rénale chronique, de troubles de la fonction intestinale, de l'hypertension, de l'hypertonie essentielle, de maladies du système nerveux central, de maladies qui résultent d'une surexcitabilité du SNC, de l'épilepsie et de crampes déclenchées de manière centrale ou d'états d'anxiété, de dépressions et de psychoses, d'états ischémiques du système nerveux périphérique ou central ou d'une attaque, de dommages et de maladies aigus et chroniques d'organes périphériques ou de membres, qui sont provoqués par des événements d'ischémie ou de reperfusion, de l'athérosclérose, de troubles du métabolisme des graisses, de thromboses, de troubles de la fonction biliaire, d'une attaque par des ectoparasites, de maladies suite à un dysfonctionnement endothélial, de maladies dues à des protozoaires, de la malaria, à la conservation et à l'entreposage de greffes destinées à des interventions chirurgicales, à une utilisation lors d'opérations chirurgicales et de transplantations d'organes ou au traitement d'états de choc ou du diabète et des dommages diabétiques tardifs ou de maladies dans lesquelles la prolifération cellulaire représente une cause primaire ou secondaire et aux soins de santé et à la prolongation de la vie.

7. Utilisation d'un composé de formule I et/ou de ses sels pharmaceutiquement acceptables selon l'une ou plusieurs des revendications 1 à 3, seul(s) ou en combinaison avec d'autres médicaments ou substances actives pour la préparation d'un médicament destiné au traitement ou à la prophylaxie de troubles de l'effort respiratoire et/ou de troubles respiratoires provoqués par le sommeil, tels que les apnées du sommeil.

8. Utilisation d'un composé de formule I et/ou de ses sels pharmaceutiquement acceptables selon l'une ou plusieurs des revendications 1 à 3, seul(s) ou en combinaison avec d'autres médicaments ou substances actives pour la préparation d'un médicament destiné au traitement ou à la prophylaxie du ronflement.

9. Utilisation d'un composé de formule I et/ou de ses sels pharmaceutiquement acceptables selon l'une ou plusieurs des revendications 1 à 3, seul(s) ou en combinaison avec d'autres médicaments ou substances actives pour la préparation d'un médicament destiné au traitement ou à la prophylaxie de maladies rénales aiguës ou chroniques, de la défaillance rénale aiguë ou de la défaillance rénale chronique.

10. Utilisation d'un composé de formule I et/ou de ses sels pharmaceutiquement acceptables selon l'une ou plusieurs des revendications 1 à 3, seul(s) ou en combinaison avec d'autres médicaments ou substances actives pour la préparation d'un médicament destiné au traitement ou à la prophylaxie de troubles de la fonction intestinale.

11. Remède destiné à une utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 3.

12. Remède destiné à une utilisation humaine, vétérinaire ou phytoprotectrice, contenant une quantité efficace d'un composé de formule I et/ou d'un sel pharmaceutiquement acceptable de celui-ci selon l'une ou plusieurs des revendications 1 à 3, en combinaison avec une ou plusieurs autres substances actives pharmacologiques ou un ou plusieurs autres médicaments.
